# EUROPÄISCHE PATENTSCHRIFT

(11) **EP 2 002 806 B1**
(45) Veröffentlichungstag und Bekanntmachung des Hinweises auf die Patenterteilung: **14.02.2018**
(21) Anmeldenummer: 08010137.1
(22) Anmeldetag: 04.06.2008
(51) Int. Cl.: A61F 2/64, A61F 2/80, A61F 2/50, A61F 2/60

(54) **Verkleidung für eine Prothese**
Clothing for a prosthetic
Revêtement pour une prothèse

(30) Priorität: 14.06.2007 DE 202007008527 U; 28.06.2007 DE 202007009077 U
(43) Veröffentlichungstag der Anmeldung: 17.12.2008
(73) Patentinhaber: Münch, Thomas, 47167 Duisburg (DE)
(72) Erfinder: Münch, Thomas, 47167 Duisburg (DE)
(74) Vertreter: Röther, Peter

(56) Entgegenhaltungen:
- EP-A2- 0 985 388
- DE-U1- 20 309 318
- GB-A- 2 166 356
- JP-A- 2007 068 855
- US-A1- 2007 142 924

## Beschreibung

Die Erfindung betrifft eine Beinprothese gemäß dem Oberbegriff des Patentanspruchs 1.

Derartige Prothesen sind beispielsweise aus der JP 2007 068855 A sowie aus der US 2007/142924 A1 bekannt.

Hier sind im Bereich zwischen dem Oberschenkelteil und dem Unterschenkelteil einerseits Schutzvorrichtungen in Form einer künstlichen Kniescheibe verwirklicht, wobei eine derartige Ausgestaltung konstruktiv aufwendig und störungsanfällig ist. Andererseits ist zwischen dem Oberschenkelteil und dem Unterschenkelteil eine Abdeckung des Kniegelenks beschrieben, die den Zweck erfüllen soll, dass sich die Hose beim Strecken nicht einklemmt. Auch diese Ausführungsform ist für den erfindungsgemäßen Zweck nicht unbedingt geeignet.

Darüber hinaus ist aus der GB 2 166 356 A eine gattungsgemäße Prothese bekannt, bei der in einer speziellen Kniegelenkskonstruktion eine Lasche angeordnet ist, die den Spalt zwischen Oberschenkelteil und Unterschenkelteil im gebeugten Zustand verschließt und beim Strecken des Beines diese Position verlässt.

Es handelt sich bei dieser Konstruktion um ein spezielles und aufwendig herzustellendes Bauteil, das eine nachträgliche Aufrüstung vorhandener Prothesen erschwert.

Bei der vorliegenden Erfindung kann es sich um eine sogenannte Endo-Exo-Prothese handeln, bei der das Oberschenkelteil im Knochen des Stumpfes befestigt ist. Dieses Teil besteht aus einem Rohr oder Pofil, welches im Querschnitt geringer ist als der Querschnitt des Stumpfes bzw. des vorher vorhandenen Oberschenkels.

Wenn der Prothesenträger eine Hose trägt, fällt dieser Unterschied, so lange er steht, nicht auf. Sobald der Träger sich setzt, fällt die Hose zwischen Stumpf und Knie auf das Prothesenprofil, was optisch störend ist.

Ein weiterer Nachteil besteht darin, dass sich dieses Teil bei Sonneneinstrahlung aufheizt, wobei diese Wärme über die Verbindung an den Stumpf weitergeleitet wird. Dies ist unangenehm und kann auch zu anderen Problemen führen.

Der Erfindung liegt daher die Aufgabe zugrunde, den eben angesprochenen Bereich der Prothese so auszugestalten, dass zum einen ein kosmetischer Effekt und zum anderen auch ein Isolationseffekt gegeben ist.

Die Erfindung löst diese Aufgabe mit den Merkmalen des Patentanspruchs 1.

Auf diese Weise ist gewährleistet, dass, wenn der Prothesenträger sich setzt, ein Einfallen der Hose unterbleibt und es kaum auffällt, dass die Person eine Prothese trägt. Zum anderen ist gewährleistet, dass durch die Verkleidung eine direkte Wärmebeeinflussung des Prothesenteils und damit des Knochens und Stumpfes unterbunden wird. Wegen der freiliegenden Zunge ist ein Strecken- und Beugen der Prothese störungsfrei möglich.

Andere Materialien als solche auf Silikonbasis sind denkbar und werden für die Erfindung nicht ausgeschlossen.

Die Verkleidung besteht bevorzugt aus einer flexiblen Silikonplatte, die den Oberschenkelteil der Prothese und das Stumpfende von der Oberschenkelvorderseite ausgehend bis zu den Schenkel- und Kniegelenkseiten umgibt und an der Oberschenkelrückseite offen ist.

Auf diese Weise ist die Verkleidung auf einfache Weise anzubringen und auf ebenfalls einfache Weise an die Patientenmaße anzupassen.

Von Vorteil ist es, wenn die Verkleidung über das Kniegelenk hinaus verlängert und dort seitlich befestigt ist.

Auf diese Weise ist auch das künstliche Kniegelenk sowohl habituell als auch thermisch abgedeckt.

Zum besseren Tragkomfort ist dabei gemäß Anspruch 5 vorgeschlagen, dass das Silikonmaterial verschiedene Shorehärten aufweist, derart, dass das Material im Kniebereich weicher ist als das Material im Stumpfbereich.

Somit ist gewährleistet, dass der Bereich vom Stumpfende bis vor das Knie ausreichend steif ist, wodurch das Einfallen der Hose verhindert wird, der Bereich des Knies aber so elastisch, dass die Bewegung des Unterschenkelbereichs gegenüber dem Oberschenkelbereich möglichst wenig gestört wird. Dies trifft insbesondere für ein so genanntes C-Leg zu, bei dem die Gehbewegung computer- und ventilgesteuert unterstützt wird.

Die Erfindung wird im folgenden anhand der Zeichnungen dargestellt und erläutert.

Es zeigen:
- Fig. 1: eine Prinzipskizze der verkleideten Prothese in seitlicher Anschauung
- Fig. 2: Verkleidung in Seitenansicht
- Fig. 3: Vorderansicht der Verkleidung

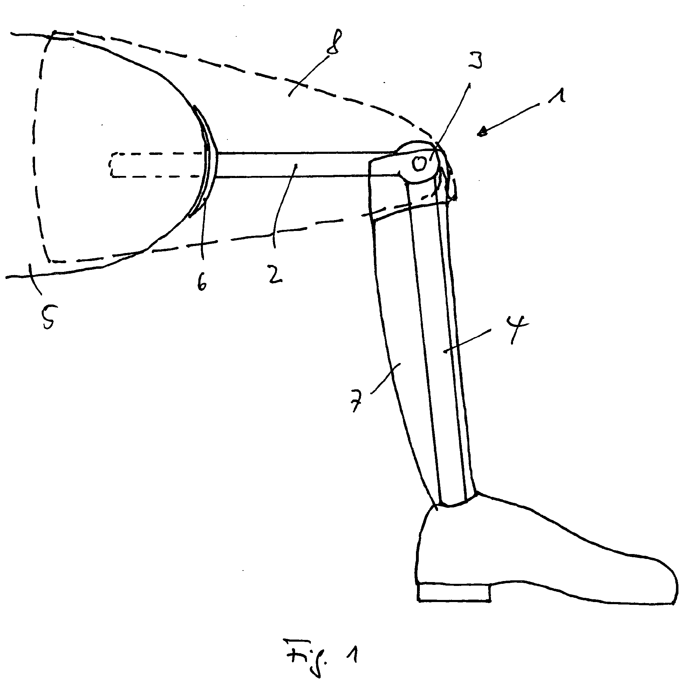

In der Figur 1 ist eine so genannte Endo-Exo-Beinprothese dargestellt und allgemein mit dem Bezugszeichen 1 versehen. Sie besteht aus einem Oberschenkteil 2, das über ein Gelenk 3 mit dem Unterschenkelteil 4 verbunden ist. Das Oberschenkelteil 2 ist am Stumpf 5 des beinamputierten Patienten durch Verankerung im Knochen des Stumpfes 5 befestigt. Mit dem Bezugszeichen 6 ist eine Schutzkappe bezeichnet.

Das Unterschenkelteil 4 ist umgeben von einer im vorliegenden Falle durchsichtigen, wadenähnlichen Abdeckung 7.

Bei der dargestellten Prothese kann es sich um ein so genanntes C-Leg handeln, welches computer- und ventilgesteuert den Bewegungsablauf beim Gehvorgang unterstützt.

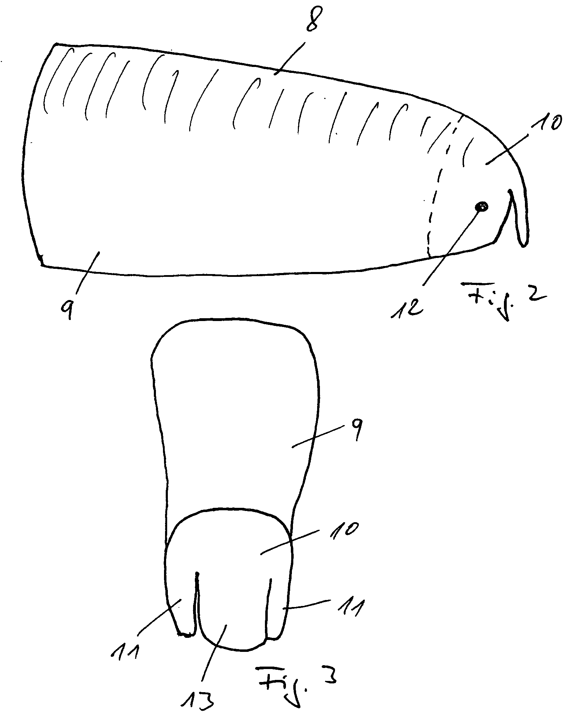

Vom Stumpf 5 bis zum Kniegelenk 3 umgibt den Oberschenkelteil 2 eine Abdeckung 8 aus Silikon, die in der Figur 2 deutlicher dargestellt ist.

Die Abdeckung 8 besteht wie gesagt aus einem Silikonlappen, bei dem das Silikonmaterial zwei Shorehärten aufweist.

Im Bereich 9 ist das Material härter als im Bereich 10. Durch das härtere Material im Bereich 9 ist gewährleistet, dass sich die Abdeckung 8 sicher um den Stumpf legt und vor allen Dingen beim Sitzen des Patienten verhindert, dass die Hose bis auf das Oberschenkelteil 2 der Prothese absinkt.

Das weichere Material im Bereich 10 gewährleistet, dass beim Beugen und Strecken des Unterschenkelteils 4 der Kniegelenkbereich 3 immer abgedeckt ist, ohne dass das Material hier stört.

Aus diesem Grunde ist wie in Figur 3 dargestellt, dieser Bereich 10 so aufgebaut, dass seitlich links und rechts zwei Wangen 11 vorhanden sind, in denen sich die Befestigungen 12 befinden, mit denen die Abdeckung 8 im Kniebereich der Prothese befestigt wird.

Zwischen den Wangen 11 befindet sich eine den Kniebereich nach vorne abdeckende Zunge 13.

Die Abdeckung 8 umgibt den Stumpf 5 nur zum Teil, ist also im rückwärtigen Schenkelbereich offen.

Die Abdeckung 8 hat dabei durch ihre Form und Stützfunktion für die Hose des Prothesenträgers nicht nur einen kosmetischen Zweck sondern darüber hinaus auch die Aufgabe, Aufheizungen der Prothese im Oberschenkelteil 2 durch Sonneneinstrahlung zu verhindern, so dass eine Wärmeleitung über den Teil 2 in den Stumpf 5 ausgeschlossen ist.

## Patentansprüche

1. Beinprothese bestehend aus einem Unterschenkelteil mit daran angeordnetem Fußteil und einem Oberschenkelteil, das kniegelenkartig an dem Unterschenkelteil angelenkt ist, wobei das Oberschenkelteil (2) vom Kniegelenk (3) bis zum Stumpfende (5) mit einer Verkleidung (8) auf insbesondere Silikonbasis versehen ist, die konisch vom Stumpf (5) bis zum Kniegelenk (3) zu läuft,
***dadurch gekennzeichnet,***
**dass** der Kniebereich (10) der Verkleidung (8) aus einer linken und einer rechten, an der Prothese befestigten Seitenwange (11) und einer zwischen diesen angeordneten, aber seitlich von ihnen getrennten, in Richtung Unterschenkelteil (4) verlaufenden Zunge (13) besteht, die die Verkleidung (8) über das Kniegelenk (3) hinaus verlängert.

2. Beinprothese nach Anspruch 1,
**dadurch gekennzeichnet,**
**dass** die Verkleidung (8) den Oberschenkelteil (2) und das Stumpfende (5) von der Oberschenkelvorderseite ausgehend bis zu den Schenkel- und Kniegelenkseiten umgibt und an der Oberschenkelrückseite offen ist.

3. Beinprothese nach Anspruch 1 oder 2,
**dadurch gekennzeichnet,**
**dass** das Oberschenkelteil (2) der Prothese (1) mittels einer osteointegrierten Halterung mit dem Stumpfknochen verbunden ist.

4. Beinprothese nach einem der Ansprüche 1 bis 3,
**dadurch gekennzeichnet,**
**dass** es sich um eine Oberschaft-Prothese handelt.

5. Beinprothese nach einem der Ansprüche 1 bis 4,
**dadurch gekennzeichnet,**
**dass** das Silikonmaterial verschiedene Shorehärten aufweist, wobei das Material im Kniebereich (10) weicher ist als das Material im Stumpfbereich (9).

## Claims

1. Leg prosthesis composed of a lower leg part, with a foot part arranged on the latter, and of an upper leg part which is articulated on the lower leg part in the manner of a knee joint, the upper leg part (2) being provided with a covering (8), particularly a silicone-based covering (8), from the knee joint (3) as far as the stump end (5), which covering (8) tapers conically from the stump (5) to the knee joint (3), **characterized in that** the knee area (10) of the covering (8) is composed of a left-hand and a right-hand side panel (11) secured on the prosthesis and of a tongue (13) which is arranged between the side panels (11) but which extends laterally separate from them in the direction of the lower leg part (4), which tongue (13) continues the covering (8) beyond the knee joint (3).

2. Leg prosthesis according to Claim 1, **characterized in that** the covering (8) surrounds the upper leg part (2) and the stump end (5) from the front of the upper leg as far as the thigh and knee-joint sides and is open at the rear of the upper leg.

3. Leg prosthesis according to Claim 1 or 2, **characterized in that** the upper leg part (2) of the prosthesis (1) is connected to the stump bone by means of an osseointegrated holder.

4. Leg prosthesis according to one of Claims 1 to 3, **characterized in that** it is a transfemoral prosthesis.

5. Leg prosthesis according to one of Claims 1 to 4, **characterized in that** the silicone material has different degrees of Shore hardness, the material in the knee area (10) being softer than the material in the stump area (9).

## Revendications

1. Prothèse de jambe, constituée d'une partie de jambe inférieure avec une partie de pied disposée sur celle-ci et d'une partie de cuisse qui est articulée à la manière d'une articulation de genou à la partie de jambe inférieure, la partie de cuisse (2), depuis l'articulation du genou (3) jusqu'à l'extrémité du moignon (5), étant pourvue d'un habillage (8) notamment à base de silicone, qui s'étend sous forme conique depuis le moignon (5) jusqu'à l'articulation du genou (3),
**caractérisée en ce que**
la région du genou (10) de l'habillage (8) se compose d'une joue latérale (11) gauche et droite, fixée à la prothèse, et d'une langue (13) disposée entre celles-ci, mais séparée latéralement de celles-ci, s'étendant dans la direction de la partie de jambe inférieure (4), laquelle langue prolonge l'habillage (8) au-delà de l'articulation du genou (3).

2. Prothèse de jambe selon la revendication 1,
**caractérisée en ce que**
l'habillage (8) entoure la partie de cuisse (2) et l'extrémité de moignon (5) depuis le côté avant de la cuisse jusqu'aux côtés d'articulation entre la cuisse et le genou, et est ouvert au niveau du côté arrière de la cuisse.

3. Prothèse de jambe selon la revendication 1 ou 2,
**caractérisée en ce que**
la partie de cuisse (2) de la prothèse (1) est reliée à l'os du moignon au moyen d'une fixation ostéo-intégrée.

4. Prothèse de jambe selon l'une quelconque des revendications 1 à 3,
**caractérisée en ce**
**qu'**il s'agit d'une prothèse à emboîture.

5. Prothèse de jambe selon l'une quelconque des revendications 1 à 4,
**caractérisée en ce que**
le matériau en silicone présente différentes duretés Shore, le matériau dans la région du genou (10) étant plus souple que le matériau dans la région du moignon (9).
